# EUROPÄISCHE PATENTANMELDUNG

(11) **EP 4 653 101 A2**
(43) Veröffentlichungstag der Anmeldung: **26.11.2025**
(21) Anmeldenummer: 25208457.9
(22) Anmeldetag: 28.05.2021
(51) Int. Cl.: B08B 9/00

(54) **REINIGUNGSVORRICHTUNG FÜR EIN EINEN UNTERSUCHUNGSTUNNEL AUFWEISENDES TOMOGRAPHIEGERÄT UND TOMOGRAPHIEGERÄT**

(30) Priorität: 27.06.2020 DE 102020207984
(62) Teilanmeldung aus: 21730169.6
(71) Anmelder: Radiology Innovations NB20C GmbH, 91088 Bubenreuth (DE)
(72) Erfinder: NAGEL, Armin Michael, 91088 Bubenreuth (DE); BICKELHAUPT, Sebastian, 91052 Erlangen (DE)
(74) Vertreter: FDST Patentanwälte

(57) **Zusammenfassung**

Die Erfindung betrifft eine Reinigungsvorrichtung (10) für ein einen Untersuchungstunnel (4) aufweisendes Tomographiegerät (1), insbesondere ein MRT. Diese Reinigungsvorrichtung (10) weist eine Düsenanordnung (12) zur Applikation eines flüssigen Reinigungsmittels auf eine zur Tunnelachse weisende Tunnelinnenfläche (8), eine Haltevorrichtung, die dazu eingerichtet ist, die Düsenanordnung (12) parallel zur Tunnelachse und entlang des Untersuchungstunnels (4) verschiebbar an einer Baueinheit (6) des Tomographiegeräts (1) zu halten, und ein Steuergerät (18) auf, das dazu eingerichtet ist, im bestimmungsgemäßen Reinigungsbetrieb die Applikation des Reinigungsmittels in Abhängigkeit von der Bewegung der Düsenanordnung (12) vorzugeben.

## Beschreibung

Die Erfindung betrifft eine Reinigungsvorrichtung für ein einen Untersuchungstunnel aufweisendes Tomographiegerät. Des Weiteren betrifft die Erfindung ein solches Tomographiegerät.

Tomographiegeräte dienen, wie der Name bereits angibt, insbesondere zur Erstellung von Schnittbildern eines Untersuchungsobjekts, bspw. eines Patienten. Eine Variante zur Erstellung der Schnittbilder ist die Nutzung von Röntgenstrahlung, bspw. bei einem Computertomographiegerät (kurz: CT). Eine weitere Variante ist die sogenannte Magnetresonanztomographie (auch als Kernspintomographie bezeichnet), die bei einem Magnetresonanztomographiegerät (kurz: MRT) zum Einsatz kommt. Es sind darüber hinaus auch Kombinationsgeräte bekannt, bspw. ein Positronen-Emissions-MRT (kurz: PET-MRT), ein PET-CT und dergleichen. Üblicherweise weisen Tomographiegeräte einen Untersuchungstunnel auf, in den das Untersuchungsobjekt hineingeschoben wird. Bei einem Computertomographiegerät wird dieser Untersuchungstunnel durch die sogenannte Gantry gebildet, die wiederum an einem Drehkranz gehaltert eine Röntgenstrahlenquelle sowie üblicherweise in Gegenüberstellung einen Röntgenstrahlendetektor trägt. Bei einem auf Magnetresonanztomographie (kurz: MR) basierenden Gerät wird der Untersuchungstunnel durch die ringförmig angeordneten Magnetspulen gebildet.

Insbesondere bei der Untersuchung von Patienten ist eine Reinigung der Oberflächen der Tomographiegeräte wichtig, um Schmierinfektionen bei Berührung der Oberflächen durch die Patienten zu vermeiden. Gerade bei MRTs sind jedoch die Untersuchungstunnel (im Hinblick auf ihren Durchmesser) vergleichsweise klein - üblicherweise im Bereich um 60 bis 70 Zentimeter - sowie häufig auch vergleichsweise langgestreckt. So sind bei MR-basierten Tomographiegeräten (insbesondere zur Ganzkörperuntersuchung) Tunnellängen von über 1 Meter üblich und auch Längen von mehr als 1,6 bis zu knapp 3 Meter bekannt. Diese Tunnellänge zusammen mit dem vergleichsweise geringen Durchmesser ergibt erkanntermaßen eine vergleichsweise schlechte Zugänglichkeit der Innenseite des Untersuchungstunnel, der "Tunnelinnenfläche".

Dazu kommt, dass moderne MRTs bereits magnetische Feldstärken von mehr als 2 Tesla, teilweise sogar um 7 Tesla aufweisen. Diese Feldstärken werden üblicherweise von supraleitenden Magnetspulen erzeugt, die aber auch bei Trennung von der Energiezufuhr stromführend und somit magnetisch sind. Aufgrund von Arbeitsschutzbestimmungen darf Personal aber nicht magnetischen Feldstärken größer 2 Tesla oder einer vergleichbaren, durch eine zeitliche Änderung der magnetischen Flussdichte (bspw. bei Bewegung im Streufeld) hervorgerufenen Exposition ausgesetzt werden.

Aus diesem Grund erfolgt eine Reinigung von MRTs häufig nur in vergleichsweise großen zeitlichen Intervallen und unter hohem (unter anderem zeitlichen) Aufwand, was im klinischen Betrieb aber bspw. aufgrund von Hygieneanforderungen und/oder Taktung von Untersuchungsterminen problematisch sein kann.

WO 2010/146482 A1 beschreibt einen Lastkraftwagen, auf dessen Ladefläche eine Isolations-Röhre sowie eine Untersuchungseinheit, bspw. ein CT oder ein MRT, angeordnet ist. Für eine Dekontamination der Isolations-Röhre wird eine Sprühapplikation eines Reinigungsmittels beschrieben.

US 2012/195410 A1 beschreibt ein CT, bei dem eine Reinigungsvorrichtung außerhalb des Röntgenstrahlen-Bereichs insbesondere während einer Bilderfassung erfolgt.

US 5,918,342 A beschreibt eine Art handgeführten Wischmop für die Reinigung von MRTs.

NORMAN BUTLER ET AL: "Device for MRI scanner intra-bore disinfection", PENN CENTER FOR INNOVATION, EMPOWERING IDEAS, PENN CENTER FOR INNOVATION, US, 23. Juni 2020 (2020-06-23), Seite 1, XP009528630, Gefunden im Internet: URL: https://upenn.technologypublisher.com/technology/40453, beschreibt die Nutzung von UV-C-Strahlung zur Desinfektion von MRTs.

Der Erfindung liegt die Aufgabe zugrunde, die Reinigung eines Tomographiegeräts, das einen Untersuchungstunnel aufweist, zu verbessern.

Diese Aufgabe wird erfindungsgemäße gelöst durch eine Reinigungsvorrichtung mit den Merkmalen des Anspruchs 1. Des Weiteren wird diese Aufgabe erfindungsgemäß gelöst durch ein Tomographiegerät mit den Merkmalen des Anspruchs 9. Vorteilhafte und teils für sich erfinderische Ausführungsformen und Weiterbildungen der Erfindung sind in den Unteransprüche sowie der nachfolgenden Beschreibung dargelegt.

Die erfindungsgemäße Reinigungsvorrichtung ist für ein einen Untersuchungstunnel aufweisendes Tomographiegerät, insbesondere ein MRT, ausgebildet und vorgesehen. Die Reinigungsvorrichtung weist gemäß einer nicht beanspruchten Ausführung dazu eine Düsenanordnung zur Applikation eines flüssigen Reinigungsmittels auf eine zur Tunnelachse weisende Tunnelinnenfläche auf. Des Weiteren weist die Reinigungsvorrichtung eine Haltevorrichtung auf, die dazu eingerichtet ist, die Düsenanordnung parallel zur Tunnelachse (also einer Achse, um die sich der Untersuchungstunnel erstreckt) und entlang des Untersuchungstunnels verschiebbar an einer Baueinheit des Tomographiegeräts zu halten.

Die Reinigungsvorrichtung ist mithin dahingehend autark ausgebildet, dass keine manuelle Applikation des flüssigen Reinigungsmittels erforderlich ist. Vorzugsweise ist die Reinigungsvorrichtung auch vollständig autark, so dass insbesondere lediglich eine Auslösung eines von der Reinigungsvorrichtung selbständig - zweckmäßigerweise von einem Steuergerät überwachten oder gesteuerten - durchgeführten Reinigungsvorgangs erforderlich ist.

Unter dem Begriff "Baueinheit" wird hier und im Folgenden insbesondere ein strukturelles Bauteil, das vorzugsweise auch von einer Außenseite des Tomographiegeräts her zugänglich ist, verstanden. Beispielsweise handelt es sich bei der Baueinheit um eine Patientenliege, auf der im bestimmungsgemäßen Betrieb des Tomographiegeräts ein Patient durch den Untersuchungstunnel verschoben wird, oder um eine Stützstruktur für diese Patientenliege.

Unter "MRT" wird hier und im Folgenden insbesondere sowohl ein "reines" Magnetresonanztomographiegerät als auch ein auch auf der Nutzung von Magnetresonanz basierendes Kombinationsgerät verstanden, bspw. ein PET-MRT oder ein MR-LINAC (letzteres umfasst einen Linearbeschleuniger zur Strahlentherapie).

Die Erfindung bietet den Vorteil, dass kein Reinigungspersonal sich in den Untersuchungstunnel begeben braucht, sowie dass, insbesondere aufgrund der Düsenanordnung, eine flächendeckende Reinigungsmittelapplikation mit (im Vergleich zu einer manuellen Aufbringung) hoher Wiederholgenauigkeit ermöglicht wird.

Vorzugsweise kommt als flüssiges Reinigungsmittel ein Desinfektionsmittel oder eine Kombination aus einem "typischen" Reinigungsmittel (das üblicherweise waschaktive Substanzen, bspw. Tenside enthält) und einem "typischen" Desinfektionsmittel (das üblicherweise zum überwiegenden Teil aus einem Alkohol oder dergleichen gebildet ist) zum Einsatz.

Konkret hält die Haltevorrichtung die Düsenanordnung im bestimmungsgemäßen Einsatzzustand an einer Patientenliege des Tomographiegeräts vorzugweise derart, dass die Düsenanordnung bei Bewegung der Patientenliege mitbewegt wird. Anders ausgedrückt ist die Düsenanordnung mittels der Haltevorrichtung an der Patientenliege befestigt.

In einer alternativen Ausführung weist die Haltevorrichtung eine Schienenführung auf. Mittels dieser ist die Düsenanordnung dabei separat von der Patientenliege in dem Untersuchungstunnel verschiebbar geführt.

In einer zweckmäßigen Weiterbildung weist die Haltevorrichtung dabei einen Schienengleiter auf, der im bestimmungsgemäßen Einsatzzustand mit einer Führungsschiene der Patientenliege koppelt. In diesem Fall ist die Reinigungsvorrichtung, insbesondere die Düsenanordnung im bestimmungsgemäßen Einsatzzustand also verschiebbar an einer Führungsschiene der Patientenliege geführt. Dadurch lässt sich die Düsenanordnung unabhängig von der Patientenliege bewegen.

Im Fall der eigenen Schienenführung weist die Reinigungsvorrichtung vorzugsweise auch einen Antrieb auf, der die Bewegung der Düsenanordnung entlang der Schienenführung und somit durch den Untersuchungstunnel bewirkt.

In einer optionalen Ausführung ist die Düsenanordnung durch einen Düsenkopf mit einer Mehrzahl von Düsenöffnungen gebildet. Diese sind relativ zur Bewegungsachse des Düsenkopfs in unterschiedliche Radialrichtungen gerichtet. Die dadurch hervorgerufene Sprühgeometrie ist dabei derart gestaltet, dass die zu reinigende Tunnelinnenfläche flächig (d. h. zumindest in einer Radialebene linienförmig) besprüht werden kann. Der Düsenkopf ist dabei beispielsweise auf der Tunnelachse angeordnet. Beispielsweise weist der Düsenkopf einen Durchmesser von etwa 10 Zentimeter auf.

In einer bevorzugten Ausführung weist die Düsenanordnung einen Haltebügel (oder auch: Halterahmen) auf, an dem eine Mehrzahl von Einzeldüsen angeordnet ist. Diese Einzeldüsen sind, vergleichbar zu dem Düsenkopf, insbesondere entlang dem Haltebügel verteilt und vorzugsweise in unterschiedliche Radialrichtungen gerichtet. Dadurch wird wiederum die vorstehend beschriebene Sprühgeometrie ausgebildet. Im bestimmungsgemäßen Einsatzzustand sind die Einzeldüsen dabei außerdem zweckmäßigerweise mit vergleichsweise geringem Abstand zur Tunnelinnenfläche angeordnet.

In einer zweckmäßigen Variante ist der Haltebügel als Kreis oder Kreisbogenabschnitt ausgebildet. Als Kreisbogenabschnitt ist der Haltebügel insbesondere für den Fall ausgebildet, dass der Untersuchungstunnel auf seiner Unterseite abgeflacht ist und eine integrierte Führungsstruktur für die Patientenliege aufweist. In diesem Fall wird vorzugsweise lediglich der durch die Patientenliege im bestimmungsgemäßen Betrieb nicht verdeckte Flächenbereich des Untersuchungstunnels, der für den Patienten zugänglich ist, mit Reinigungsmittel beaufschlagt. Der Haltebügel ist in diesem Fall also vorzugsweise der (zumindest für den Patienten zugänglichen Bereich der) Innenkontur des Untersuchungstunnels nachgeformt. Dadurch wird vorteilhafterweise ermöglicht, die Einzeldüsen mit jeweils zumindest nahezu dem gleichen Abstand zu der Tunnelinnenfläche anzuordnen.

In einer alternativen Variante ist der Haltebügel polygonal, bspw. als Rechteck, ausgebildet.

Erfindungsgemäß weist die Reinigungsvorrichtung (folglich alternativ oder optional zusätzlich zu der Düsenanordnung) eine Wischeranordnung auf. Diese ist dazu eingerichtet, die Tunnelinnenfläche mechanisch unter Wirkung des (flüssigen) Reinigungsmittels zu reinigen und/oder - insbesondere im Fall der Kombination mit der Düsenanordnung - das Reinigungsmittel wieder aufzunehmen. Dazu trägt die Wischeranordnung vorzugsweise eine Anzahl von mechanischen Reinigungselementen. Im Fall der Kombination mit der Düsenanordnung ist die Wischeranordnung vorteilhafterweise in einer bestimmungsgemäßen Vorschubrichtung der Düsenanordnung dieser nachgelagert. Anders ausgedrückt ist die Wischeranordnung hierbei so angeordnet, dass sie der Düsenanordnung nachfolgt und somit vorzugsweise das aufgebrachte, flüssige Reinigungsmittel verteilen, einarbeiten und/oder wieder aufnehmen kann. Dies ist dahingehend vorteilhaft, dass regelmäßig eine Wischdesinfektion wirkungsvoller als eine reine Sprühdesinfektion ist.

In der voranstehend beschriebenen, für sich alleine erfinderischen Ausführung kann die Düsenanordnung bei der Reinigungsvorrichtung auch nicht vorhanden sein. Die Haltevorrichtung ist in diesem Fall dazu eingerichtet, die Wischeranordnung parallel zur Tunnelachse und entlang des Untersuchungstunnels verschiebbar am Tomographiegerät zu halten.

Die vorstehend mit Bezug auf die Düsenanordnung beschriebenen Merkmale der Haltevorrichtung gelten vorzugsweise entsprechend auch für die Ausführung, bei der nur die Wischeranordnung vorhanden ist.

Vorzugsweise weist das Reinigungsgerät auch ein Steuergerät (insbesondere das vorstehend erwähnte) auf, das dazu eingerichtet ist, im bestimmungsgemäßen Reinigungsbetrieb die Bewegung (bspw. die Vorschubgeschwindigkeit) der Düsenanordnung und/oder der Wischeranordnung durch den Untersuchungstunnel vorzugeben und somit den Reinigungsvorgang zu steuern. Alternativ oder zusätzlich ist das Steuergerät dazu eingerichtet, die Applikation des Reinigungsmittels in Abhängigkeit von der Bewegung der Düsenanordnung (relativ zum Tomographiegerät) und/oder von der Bewegung der Wischeranordnung vorzugeben. Die "Applikation des Reinigungsmittels" ist hierbei insbesondere derart zu verstehen, dass diese sowohl durch Aufsprühen mittels der Düsenanordnung als auch durch mechanischen Auftrag, bspw. mittels getränkter Reinigungselemente der Wischeranordnung, erfolgen kann.

Unter "in Abhängigkeit von der Bewegung der Düsenanordnung" wird hier und im Folgenden insbesondere verstanden, dass die Applikation des Reinigungsmittels in Abhängigkeit von der Position der Düsenanordnung im Untersuchungstunnel und/oder von einer Vorschubgeschwindigkeit der Düsenanordnung bei deren Bewegung durch den Untersuchungstunnel vorgegeben wird. Die Positionsabhängigkeit kann bspw. bei einer unregelmäßigen Oberfläche der Tunnelinnenfläche vorteilhaft sein, so dass an manchen Stellen mehr Reinigungsmittel aufgebracht werden muss. Die Geschwindigkeitsabhängigkeit wird bspw. dadurch realisiert, dass bei einer hohen Vorschubgeschwindigkeit ein vergleichsweise höherer Reinigungsmitteldurchfluss vorgegeben wird als bei einer geringen Vorschubgeschwindigkeit, insbesondere derart, dass stets ein (hinsichtlich der Menge des Reinigungsmittels) möglichst konstanter Auftrag pro Flächeneinheit erfolgt.

In einer zweckmäßigen Ausführung - insbesondere für den Fall, dass die (insbesondere separate) Düsenanordnung nicht vorhanden ist - ist das Reinigungselement, vorzugsweise jedes von gegebenenfalls mehreren der Reinigungselemente, der Wischeranordnung mit einer Reinigungsmittelzuführung, bspw. einer am entsprechenden Reinigungselement mündenden Leitung (insbesondere ein Schlauch oder dergleichen) gekoppelt, so dass das entsprechende Reinigungselement, in diesem Fall vorzugsweise ein Schwamm, eine Bürste, ein Tuch oder dergleichen, (insbesondere automatisch) mit dem Reinigungsmittel zur Applikation des Reinigungsmittels getränkt werden kann. Grundsätzlich ist es aber auch möglich und optional auch so ausgeführt, dass im bestimmungsgemäßen Betrieb das entsprechende Reinigungsmittel manuell getränkt wird, vorzugsweise bevor die Wischeranordnung in den Untersuchungstunnel einfährt.

Im vorstehenden Fall, in dem das entsprechende Reinigungselement mittels der an diesem mündenden Leitung getränkt wird, ist die Mündung der Leitung zweckmäßigerweise in Form einer Art Düse so ausgebildet, dass das Reinigungselement hinreichend feucht gehalten werden kann, um einen kontinuierliche Auftrag des Reinigungsmittel auf die Tunnelinnenfläche zu ermöglichen. Dabei kann eine Art Breitschlitzdüse oder aber auch eine Art Perlator gewählt sein, um das Reinigungsmittel möglichst großflächig und/oder für eine möglichst vollständige Durchtränkung oder Benetzung in das entsprechende Reinigungselement einleiten zu können.

Das Steuergerät ist vorzugsweise zumindest im Kern durch einen Mikrocontroller mit einem Prozessor und einem Datenspeicher gebildet, in dem die Funktionalität zur Durchführung des Reinigungsvorgangs in Form einer Betriebssoftware (Firmware) programmtechnisch implementiert ist. Der Reinigungsvorgang kann dabei zweckmäßigerweise bei Ausführung der Betriebssoftware in dem Mikrocontroller - gegebenenfalls in Interaktion mit einer Bedienperson - automatisch durchgeführt werden. Optional ist diese Betriebssoftware in ein übergeordnetes Steuergerät - bspw. des Tomographiegeräts - integriert, so dass das Steuergerät der Reinigungsvorrichtung durch das Steuergerät des Tomographiegeräts gebildet ist. Das Steuergerät kann im Rahmen der Erfindung auch durch ein nicht-programmierbares elektronisches Bauteil, z.B. einen ASIC, gebildet sein, in dem die Funktionalität zur Durchführung des Reinigungsvorgangs mit schaltungstechnischen Mitteln implementiert ist. Alternativ ist im Rahmen der Erfindung auch eine Ausführung denkbar und vorgesehen, in der das Steuergerät mechanisch, hydraulisch oder pneumatisch ausgeführt ist. In diesem Fall erfolgt zweckmäßigerweise die Applikation des Reinigungsmittels getrieben von der (insbesondere Vorschub-) Bewegung der Düsenanordnung bzw. der Wischeranordnung, bspw. mittels eines Bewegungsaufnehmers (bspw. einem Reibrad, einer Schubstange, einem Stößel oder dergleichen), der im bestimmungsgemäßen Einsatzzustand mit dem Tomographiegerät in Kontakt steht, und die Ausbringung des Reinigungsmittels und/oder die Bewegung der Wischeranordnung bewirkt. Optional kann hierbei die Bewegung der Düsen- bzw. Wischeranordnung auch manuell (insbesondere durch ein Verschieben der Düsen- und/oder Wischeranordnung im Untersuchungstunnel) durch einen Bediener erfolgen.

In einer zweckmäßigen Ausgestaltung, insbesondere für den Fall, dass die Haltevorrichtung die Düsenanordnung bzw. die Wischeranordnung im bestimmungsgemäßen Einsatzzustand an einer Patientenliege des Tomographiegeräts hält, ist das Steuergerät in das (insbesondere übergeordnete) Steuergerät des Tomographiegeräts integriert.

Weiter ist der optional vorhandene Antrieb der der Reinigungsvorrichtung zugeordneten Schienenführung zweckmäßigerweise steuerungstechnisch mit dem Steuergerät verknüpft.

Optional ist der Abstand (in Vorschubrichtung) zwischen der Düsenanordnung und der Wischeranordnung dabei so gewählt, dass bei einer vorgegebenen Vorschubgeschwindigkeit eine hinreichend lange Einwirkzeit des flüssigen Reinigungsmittels gegeben ist. Zusätzlich oder alternativ ist das Steuergerät dazu ausgelegt und eingerichtet, die Vorschubgeschwindigkeit in Abhängigkeit von dem Abstand und einer empfohlenen Einwirkzeit - die häufig abhängig vom eingesetzten Typ des Reinigungsmittels ist - unterschiedlich vorzugeben.

Bevorzugt handelt es sich bei der Wischeranordnung um einen zweiten (bspw. runden oder polygonalen) Haltebügel, der insbesondere gemeinsam mit der Düsenanordnung geführt ist. An diesem sind wiederum als Reinigungselemente Schwämme, Tücher, Bürsten und/oder Abziehlippen (umgangssprachlich auch als "Gummilippen" bezeichnet) angeordnet, die zur Verteilung bzw. Aufnahme des Reinigungsmittels, insbesondere zur Wischdesinfektion geeignet sind. In einer optionalen Variante sind dabei Schwämme, Tücher oder Bürsten - gegebenenfalls mit der vorstehend beschriebenen Reinigungsmittelzuführung gekoppelt - in Vorschub- (oder auch: Einschub-) Richtung vor den Abziehlippen (oder optional auch nur einer, der Kontur der Tunnelinnenfläche nachgeformten Abziehlippe) angeordnet.

In einer weiteren optionalen Ausführung der Düsenanordnung - die auch dann zum Einsatz kommen kann, wenn die Wischeranordnung vorhanden ist - ist diese nicht wie vorstehend beschrieben zur Sprühapplikation des Reinigungsmittels ausgebildet, sondern, vergleichbar zur vorstehend beschriebenen Variante der Wischeranordnung zur "mechanischen" Applikation. Dazu umfasst die Düsenanordnung ein Applikationselement oder optional mehrere Applikationselemente, das oder die den Düsen der Düsenanordnung und der Tunnelinnenfläche zwischengeschaltet sind. Bei dem jeweiligen Applikationselement handelt es sich bspw. um einen Schwammkörper oder dergleichen. Dieser wird im bestimmungsgemäßen Betrieb mittels der jeweiligen Düse "rückseitig" mit dem Reinigungsmittel getränkt und kann dieses frontseitig durch Berührung der Tunnelinnenfläche auf diese auftragen. In diesem Fall ist die jeweilige Düse der Düsenanordnung also zur Übergabe des Reinigungsmittels an das Applikationselement eingerichtet. Im Fall der vorstehend beschriebenen Sprühapplikation ist die jeweilige Düse hingegen derart ausgebildet, dass die vorstehend beschriebene Sprühgeometrie auf der Tunnelinnenfläche erreicht wird.

In einer zweckmäßigen Weiterbildung weist die Wischeranordnung einen Antrieb zur von der Düsenanordnung entkoppelten Bewegung der mechanisch auf die Tunnelinnenfläche wirkenden Reinigungselemente auf. Beispielsweise umfasst dieser Antrieb einen Elektromotor. Alternativ ist der Antrieb durch ein Getriebe, eine Kulissenführung oder dergleichen gebildet, das bzw. die bspw. mit der vorstehend beschriebenen Schienenführung (oder einer Teilfläche des Reinigungstunnels, bspw. mittels eines Reibrads) derart zusammenwirkt, dass bei einer Vorschubbewegung die Reinigungselemente quer zur Vorschubbewegung bewegt werden.

In einer vorteilhaften Ausführung umfasst die Reinigungsvorrichtung einen Vorratsbehälter und gegebenenfalls auch ein Auffangbehälter für das Reinigungsmittel. Im bestimmungsgemäßen Reinigungsbetrieb ist dieser bzw. sind diese vorzugsweise außerhalb des Untersuchungstunnels angeordnet. Dazu umfasst die Reinigungsvorrichtung entsprechende Verbindungsmittel, insbesondere einen zugeordneten Schlauch, um bspw. den Vorratsbehälter mit der Düsenanordnung zu verbinden.

Für den Fall, dass ein Auffangbehälter vorhanden ist, ist zweckmäßigerweise an der Wischeranordnung eine Auffangrinne oder dergleichen angeordnet, in die das insbesondere von Abziehlippe ablaufende Reinigungsmittel einlaufen und von dieser zum Auffangbehälter fließen kann. Optional ist in diesem Fall auch eine Absaugvorrichtung vorhanden, die das Reinigungsmittel "aufsagen" kann.

In einer besonders bevorzugten Ausführung sind Elemente der Reinigungsvorrichtung, die im bestimmungsgemäßen Reinigungsbetrieb in den Untersuchungstunnel einfahren, unter Entfall oder zumindest Reduktion von magnetischem, insbesondere ferromagnetischem Material ausgebildet. Insbesondere sind solche Elemente die Düsenanordnung, die gegebenenfalls vorhandene Wischeranordnung, die Haltevorrichtung sowie gegebenenfalls vorhandene Leitungen für das Reinigungsmittel. Dadurch kann vorteilhafterweise verhindert werden, dass diese Elemente beim Einsatz der Reinigungsvorrichtung bei einem MRT von den darin verwendeten Magnetspulen angezogen werden. Somit kann die Reinigung des MRT unter Wahrung der Sicherheitsvorschriften ohne Anwesenheit von Personal in der Nähe des MRT durchgeführt werden.

In einer optionalen Ausführung weist die Reinigungsvorrichtung - zusätzlich oder alternativ zu der Wischeranordnung - eine UV-Strahler-Anordnung zur UV-Bestrahlung (zu Desinfektionszwecken) der Tunnelinnenfläche auf.

Das erfindungsgemäße Tomographiegerät ist vorzugsweise als MRT ausgebildet. Das Tomographiegerät weist einen Untersuchungstunnel sowie die vorstehend beschriebene Reinigungsvorrichtung auf. Mithin weist das Tomographiegerät die gleichen Merkmale und somit auch die gleichen Vorteile wie die Reinigungsvorrichtung auf.

Die Konjunktion "und/oder" ist hier und im Folgenden insbesondere derart zu verstehen, dass die mittels dieser Konjunktion verknüpften Merkmale sowohl gemeinsam als auch als Alternativen zueinander ausgebildet sein können.

Nachfolgend werden Ausführungsbeispiele der Erfindung anhand einer Zeichnung näher erläutert. Darin zeigen:
- Fig. 1: in einer schematischen Ansicht ein Tomographiegerät mit einer Reinigungsvorrichtung,
- Fig. 2: in Ansicht gemäß Fig. 1 ein weiteres Ausführungsbeispiel der Reinigungsvorrichtung, und
- Fig. 3: in einer schematischen Seitenansicht eine Patientenliege mit einem weiteren Ausführungsbeispiel der Reinigungsvorrichtung.

Einander entsprechende Teile sind in allen Figuren stets mit gleichen Bezugszeichen versehen.

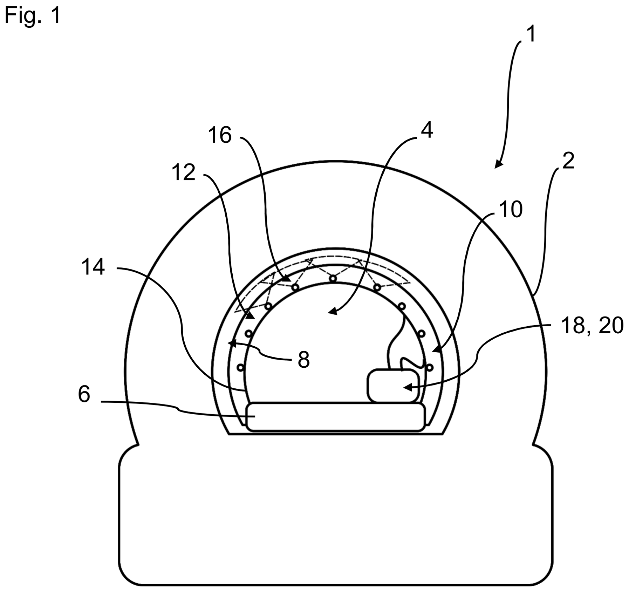

In Fig. 1 ist schematisch ein Tomographiegerät, konkret ein Magnetresonanztomographiegerät, kurz "MRT 1" dargestellt. Das MRT 1 weist ein Gehäuse 2 auf, in dem ringförmig Magnetspulen angeordnet sind, so dass ein in deren Zentrum liegender Untersuchungstunnel, kurz "Tunnel 4", gebildet wird. Durch diesen Tunnel 4 wird im bestimmungsgemäßen Untersuchungsbetrieb ein Patient mittels einer an dem Gehäuse 2 geführten Patientenliege 6 bewegt.

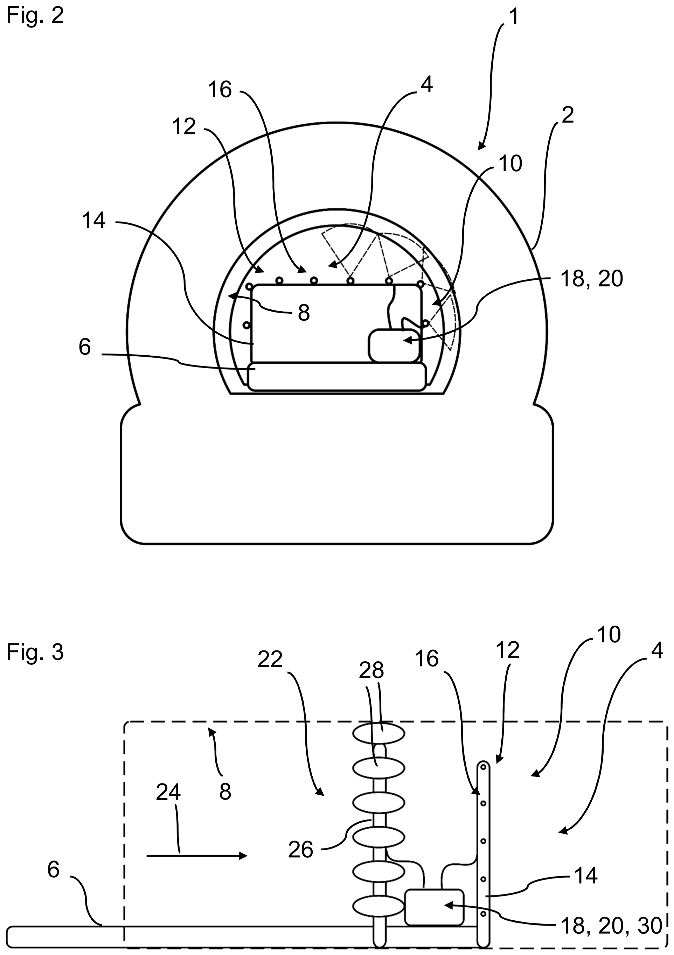

Ein Teil einer den Tunnel 4 umrandenden Tunnelinnenfläche 8 ist dabei für den auf der Patientenliege 6 liegenden Patienten zugänglich. Aus diesem Grund ist es zweckmäßig, diesen Teil der Tunnelinnenfläche 8 regelmäßig zu reinigen, insbesondere zu desinfizieren. Problematisch ist dabei - wie insbesondere anhand von Fig. 3 zu erkennen ist -, dass bei MRTs der Tunnel 4 meist vergleichsweise lang ist, häufig länger als 1,5 Meter bei einem Tunneldurchmesser von etwa 60 bis 70 Zentimeter. Dadurch ist die Zugänglichkeit zur Reinigung bereits erschwert. Dazu kommt, dass bei modernen MRTs die magnetische Feldstärke - auch im inaktiven Zustand aufgrund supraleitender Materialien - meist oberhalb der zulässigen Arbeitsplatzbelastung von 2 Tesla liegt, so dass eine Reinigung durch Reinigungspersonal nur erschwert möglich ist.

Aus diesem Grund ist dem MRT 1 eine Reinigungsvorrichtung 10 zugeordnet. Diese weist eine Düsenanordnung 12 auf, die zur Ausbringung eines flüssigen Reinigungsmittels, im Speziellen konkret eines Desinfektionsmittels, auf die Tunnelinnenfläche 8 eingerichtet ist. Dazu umfasst die Düsenanordnung 12 einen Haltebügel 14, an dem mehrere Sprühdüsen 16 (auch: "Einzeldüsen") derart angeordnet sind, dass die zu besprühende Tunnelinnenfläche 8, konkret deren zu desinfizierender Teil, flächig geschlossen besprüht werden kann. Dazu sind die Sprühdüsen 16 in jeweils unterschiedliche Radialrichtungen relativ zu einer Tunnelachse des Tunnels 4 oder Verschiebeachse der Patientenliege 6 ausgerichtet. Der Haltebügel 14 ist im vorliegenden Ausführungsbeispiel als Kreisbogenabschnitt ausgebildet, damit alle Sprühdüsen 16 (zumindest näherungsweise) den gleichen Abstand zur Tunnelinnenfläche 8 aufweisen. Die Reinigungsvorrichtung 10 weist auch eine nicht näher dargestellte Haltevorrichtung auf, mittels derer der Haltebügel 14 der Düsenanordnung 12 an der Patientenliege 6, konkret an deren Ende (s. Fig. 3) fixiert ist. Dadurch kann die Reinigungsvorrichtung 10, zumindest die Düsenanordnung 12 durch Bewegen der Patientenliege 6 durch den Tunnel 4 verfahren werden. Ein zusätzlicher Antrieb ist somit nicht erforderlich.

Des Weiteren weist die Reinigungsvorrichtung 10 ein Steuergerät 18 auf, das im dargestellten Ausführungsbeispiel als "Blackbox" auf der Patientenliege 6 dargestellt ist. Vorzugsweise ist das Steuergerät 18 aber als Softwarekomponente in ein Steuergerät des MRTs 1 integriert. In der dargestellten "Blackbox" ist auch ein Vorratsbehälter 20 für das Reinigungsmittel enthalten. Das Steuergerät 18 ist dazu eingerichtet, die Ausbringung des Reinigungsmittels in Abhängigkeit von der axialen Position der Patientenliege 6 und somit der Düsenanordnung 12 und/oder von der Vorschubgeschwindigkeit der Patientenliege 6 zu steuern.

In einer nicht näher dargestellten Ausführung ist der Vorratsbehälter 20 sowie auch eine zweckmäßigerweise zugeordnete Pumpe außerhalb des Tunnels 4 angeordnet, bspw. neben dem Gehäuse 2 und mit einer Verbindungsleitung mit den Sprühdüsen 16 gekoppelt.

In einfacher Ausführung der Reinigungsvorrichtung 10 wird lediglich eine Sprühdesinfektion der Tunnelinnenfläche 8 durchgeführt.

Damit die Teile der Reinigungsvorrichtung 10 nicht von dem Magnetfeld der Magnetspulen angezogen (oder allgemeiner ausgedrückt verschoben) werden, sind die Teile der Reinigungsvorrichtung 10, konkret die Elemente, die beim Reinigungsvorgang in den Tunnel 4 einfahren aus einem nicht-ferromagnetischen Material ausgebildet.

In Fig. 2 ist ein alternatives Ausführungsbeispiel der Reinigungsvorrichtung 10 dargestellt. Der Haltebügel 14 ist in diesem Fall etwa rechteckförmig ausgebildet. Ansonsten entspricht der Aufbau der Reinigungsvorrichtung 10 nach Fig. 1.

In Fig. 3 ist schematisch ein weiteres Ausführungsbeispiel der Reinigungsvorrichtung 10 dargestellt. Diese umfasst dabei zusätzlich zu der Düsenanordnung 12 eine Wischeranordnung 22. Diese ist in einer Vorschubrichtung 24 der Düsenanordnung 12 nacheilend angeordnet. Mit anderen Worten wird die Düsenanordnung 12 beim Einfahren der Patientenliege 6 in den Tunnel 4 zuerst in letzteren eingebracht. Die Wischeranordnung 22 weist neben einem Haltebügel 26 mehrere an letzterem gehalterte Reinigungselemente auf, hier in Form von Schwämmen 28. Diese sind entweder so angeordnet, dass eine über den zu reinigenden Teil der Tunnelinnenfläche 8 geschlossene Berührungslinie oder -fläche gebildet ist. Alternativ werden die Schwämme mittels eines nicht näher dargestellten Antriebs derart bewegt (bspw. rotiert), dass ein flächig geschlossenes Wischen dieses Teils der Tunnelinnenfläche 8 ermöglicht ist.

Beim Einfahren in den Tunnel 4 wird also zunächst die Tunnelinnenfläche 8 mittels der Sprühdüsen 16 linienförmig quer zur Vorschubrichtung 24 und damit aufgrund des Vorschubs flächig eingesprüht. Mittels der Schwämme 28 wird anschließend nach hinreichender Einwirkzeit des Reinigungsmittels letzteres aufgewischt. Die Einwirkzeit ergibt sich dabei aus der Vorschubgeschwindigkeit der Patientenliege 6 und dem Abstand zwischen Düsenanordnung 12 und Wischeranordnung 22.

Zusätzlich zu dem Vorratsbehälter 20 ist dabei auch ein Auffangbehälter 30 vorhanden, in dem mittels der Schwämme 28 aufgenommenes Reinigungsmittel aufgenommen wird. Bspw. sind die Schwämme 28 dazu mit einer Absaugvorrichtung verbunden.

In einem alternativen, nicht näher dargestellten Ausführungsbeispiel ist anstelle der Düsenanordnung 12 nur die Wischeranordnung 22 vorhanden. In diesem Fall erfolgt die Zufuhr des Reinigungsmittels in die Schwämme 28, mittels derer somit die Applikation des Reinigungsmittels erfolgt. In diesem Fall kann die Absaugvorrichtung auch entfallen und eine Trocknung an der Luft erfolgen. Ansonsten gelten die anhand von Fig. 1 und 2 beschriebenen Merkmale entsprechend übertragen auch für die Wischeranordnung 22.

Der Gegenstand der Erfindung ist nicht auf die vorstehend beschriebenen Ausführungsbeispiele beschränkt. Vielmehr können weitere Ausführungsformen der Erfindung von dem Fachmann aus der vorstehenden Beschreibung abgeleitet werden. Insbesondere können die anhand der verschiedenen Ausführungsbeispiele beschriebenen Einzelmerkmale der Erfindung und deren Ausgestaltungsvarianten auch in anderer Weise miteinander kombiniert werden.

### Bezugszeichenliste

- 1: MRT
- 2: Gehäuse
- 4: Tunnel
- 6: Patientenliege
- 8: Tunnelinnenwand
- 10: Reinigungsvorrichtung
- 12: Düsenanordnung
- 14: Haltebügel
- 16: Sprühdüse
- 18: Steuergerät
- 20: Vorratsbehälter
- 22: Wischeranordnung
- 24: Vorschubrichtung
- 26: Haltebügel
- 28: Schwamm
- 30: Auffangbehälter

## Patentansprüche

1. Reinigungsvorrichtung (10) für ein einen Untersuchungstunnel (4) aufweisendes Tomographiegerät (1), insbesondere ein MRT, aufweisend
- eine Wischeranordnung (22), die dazu eingerichtet ist, eine zur Tunnelachse weisende Tunnelinnenfläche (8) mechanisch unter Wirkung eines flüssigen Reinigungsmittels zu reinigen, und
- eine Haltevorrichtung, die dazu eingerichtet ist, die Wischeranordnung (22) parallel zur Tunnelachse und entlang des Untersuchungstunnels (4) verschiebbar an einer Baueinheit (6) des Tomographiegeräts (1) zu halten,
+ wobei die Haltevorrichtung die Wischeranordnung (22) im bestimmungsgemäßen Einsatzzustand an einer Patientenliege (6) des Tomographiegeräts (1) hält, so dass die Wischeranordnung (22) bei Bewegung der Patientenliege (6) mitbewegt wird, oder
+ wobei die Haltevorrichtung eine Schienenführung aufweist, mittels derer die Wischeranordnung (22) separat von der Patientenliege (6) in dem Untersuchungstunnel (4) verschiebbar geführt ist.

2. Reinigungsvorrichtung (10) nach Anspruch 1,
wobei die Haltevorrichtung einen Schienengleiter aufweist, der im bestimmungsgemäßen Einsatzzustand mit einer Führungsschiene der Patientenliege (6) koppelt.

3. Reinigungsvorrichtung (10) nach Anspruch 1 oder 2,
mit einem Steuergerät (18), das dazu eingerichtet ist, im bestimmungsgemäßen Reinigungsbetrieb die Applikation des Reinigungsmittels in Abhängigkeit von der Bewegung der Wischeranordnung (22) vorzugeben.

4. Reinigungsvorrichtung (10) nach einem der Ansprüche 1 bis 3,
wobei die Wischeranordnung (22) einen Haltebügel (26) umfasst, an dem eine Mehrzahl von Reinigungselemente (28) angeordnet sind.

5. Reinigungsvorrichtung (10) nach Anspruch 4,
wobei die Reinigungselemente (28) derart angeordnet sind, dass eine über den zu reinigenden Teil der Tunnelinnenfläche (8) geschlossene Berührungslinie oder -fläche gebildet ist, oder wobei die Reinigungselement (28) mittels eines Antriebs derart bewegbar sind, dass ein flächig geschlossenes Wischen dieses Teils der Tunnelinnenfläche (8) ermöglicht ist.

6. Reinigungsvorrichtung (10) nach einem der Ansprüche 1 bis 5,
wobei ein Vorratsbehälter (20) und gegebenenfalls ein Auffangbehälter (30) für das Reinigungsmittel im bestimmungsgemäßen Reinigungsbetrieb außerhalb des Untersuchungstunnels (4) verbleibt.

7. Reinigungsvorrichtung (10) nach einem der Ansprüche 1 bis 6,
wobei im bestimmungsgemäßen Reinigungsbetrieb in den Untersuchungstunnel (4) einfahrende Elemente (12, 20, 22, 30) der Reinigungsvorrichtung (10) unter Entfall oder zumindest Reduktion von ferromagnetischem Material ausgebildet sind.

8. Reinigungsvorrichtung (10) nach einem der Ansprüche 1 bis 7,
mit einer UV-Strahler-Anordnung zur UV-Bestrahlung der Tunnelinnenfläche (8).

9. Tomographiegerät (1), insbesondere MRT, aufweisend einen Untersuchungstunnel sowie eine Reinigungsvorrichtung (10) nach einem der Ansprüche 1 bis 8.
